# EUROPÄISCHE PATENTSCHRIFT

(11) **EP 2 961 731 B1**
(45) Veröffentlichungstag und Bekanntmachung des Hinweises auf die Patenterteilung: **01.02.2017**
(21) Anmeldenummer: 14706796.1
(22) Anmeldetag: 12.02.2014
(51) Int. Cl.: C07C 209/48, C07C 209/86, C07C 253/08, C07C 253/30

(54) **VERFAHREN ZUR HERSTELLUNG VON EDA UNTER VERWENDUNG VON SO2-FREIER BLAUSÄURE**
METHOD FOR PRODUCING EDA USING SO2-FREE HYDROCYANIC ACID
PROCÉDÉ DE PRODUCTION D'ÉTHYLÈNE DIAMINE EN UTILISANT DE L'ACIDE PRUSSIQUE DÉPOURVU DE SO2

(30) Priorität: 28.02.2013 EP 13157251
(43) Veröffentlichungstag der Anmeldung: 06.01.2016
(73) Patentinhaber: BASF SE, 67056 Ludwigshafen am Rhein (DE)
(72) Erfinder: LUYKEN, Hermann, 67069 Ludwigshafen (DE); JAEGLI, Stephanie, 68163 Mannheim (DE); LORENZ, Michael, 67061 Ludwigshafen (DE); BRASCHE, Gordon, 60594 Frankfurt (DE); JEGELKA, Markus, 68219 Mannheim (DE); BECKER, Barbara, 68259 Mannheim (DE); BAUMANN, Robert, 68529 Mannheim (DE); MELDER, Johann-Peter, 67459 Böhl-Iggelheim (DE); BUSCHHAUS, Boris, 69117 Heidelberg (DE); KRUG, Thomas, 67550 Worms (DE)
(74) Vertreter: BASF IP Association
(86) Internationale Anmeldenummer: PCT/EP2014/052727
(87) Internationale Veröffentlichungsnummer: WO 2014/131620

(56) Entgegenhaltungen:
- WO-A1-2004/092068
- WO-A1-2008/104552
- WO-A1-2008/104592

## Beschreibung

Die vorliegende Erfindung betrifft ein Verfahren zur Herstellung von Ethylendiamin (EDA), wobei das Verfahren die Schritte a) bis c) umfasst. In Schritt a) wird Formaldehyd mit Blausäure (HCN) zu Formaldehydcyanhydrin (FACH) umgesetzt, wobei die Blausäure vollständig frei oder weitgehend frei von Schwefeldioxid (SO₂) ist. Das auf diese Weise hergestellte FACH wird in Schritt b) mit Ammoniak (NH₃) zu Aminoacetonitril (AAN) umgesetzt, worauf in Schritt c) eine Hydrierung von AAN in Gegenwart eines Katalysators zu EDA erfolgt.

Herstellungsverfahren von EDA sind seit Langem bekannt, häufig wird hierzu Blausäure als eines der Edukte eingesetzt. WO 2008/104578 offenbart ein Verfahren zur Herstellung eines Ethylenamingemisches, das EDA enthält, wobei Roh-AAN, das weitgehend frei von Formaldehydcyanhydrin ist, bei einer Temperatur von 50 bis 150 °C erhitzt wird. Dabei wird ein Aminonitrilgemisch erhalten, das AAN und Iminodiacetonitril (IDAN) enthält. Dieses Gemisch wird anschließend in Gegenwart eines Katalysators unter Erhalt von EDA und Diethylentriamin (DETA) hydriert. WO 2008/104578 kann weiterhin entnommen werden, dass das eingesetzte Roh-AAN durch Umsetzung eines wässrigen Gemisches von Ammoniak mit FACH im Molverhältnis ≥ 4 : 1 [Mol/Mol] bei einer Temperatur von 50 bis 80 °C erhalten werden kann.

WO2008/104592 beschreibt ein Verfahren zur Herstellung von EDA durch Hydrierung von AAN, bei welchem das AAN bevorzugt durch Umsetzung von FACH mit Ammoniak hergestellt wird.

Die Herstellung von Formaldehydcyanhydrin (FACH) ist ebenfalls seit Langem bekannt. Detaillierte Informationen zur Herstellung von FACH aus Formaldehyd und Blausäure können beispielsweise WO 2008/104579 entnommen werden. In diesem Verfahren wird das FACH jedoch nicht zur Herstellung von EDA über AAN durch Umsetzung mit Ammoniak eingesetzt, stattdessen wird dort FACH mit EDA unter Erhalt von Ethylendiamindiacetonitril (EDDN) umgesetzt. EDDN kann wiederum zu Triethylentetraamin (TETA) hydriert werden.

Die zur FACH-Herstellung eingesetzte Blausäure enthält in der Praxis immer saure Stabilisatoren wie Schwefelsäure, Phosphorsäure, Essigsäure sowie insbesondere Schwefeldioxid (SO₂). Insbesondere unter dem Einfluss von Alkalien (zum Beispiel bei Aufbewahrung in Glasgefäßen) polymerisiert Blausäure langsam unter Ausscheidung schwarzbrauner Flocken bzw. durchläuft eine autokatalytische Polymerisation, was insbesondere bei großtechnischen Verfahren zur Verstopfung von Rohrleitungen führt. Durch die Zugabe von sauren Stabilisatoren, insbesondere von SO₂, wird dieses Polymerisationsverhalten von Blausäure unterbunden (siehe auch Römpp Chemielexikon, 9. Auflage 1995, Georg-Thieme-Verlag Stuttgart; sowie WO 2004/092068).

WO 2004/092068 betrifft weiterhin ein Verfahren zur Aufreinigung von Blausäure durch Destillation sowie ein Verfahren zur Hydrocyanierung von Olefinen oder Dienen. In diesem Verfahren erfolgt eine Entwässerung von Blausäure durch Destillation wässriger Roh-Blausäure mit einem HCN-Anteil von maximal 99,9 Gew.-%. Die Roh-Blausäure kann gegebenenfalls auch Kohlenstoffoxide sowie einen nicht flüchtigen Stabilisator enthalten. Die Roh-Blausäure wird in Abwesenheit eines flüchtigen Stabilisators bei einem Druck von 1 bis 2,5 bar, einer Sumpftemperatur von 100 bis 130 °C und einer Kopftemperatur von 25 bis 54 °C in einer Destillationskolonne destilliert. Aus dem Kopfabzugsstrom kann die gereinigte wasserfreie Blausäure gewonnen werden. Die gegebenenfalls enthaltenen nicht flüchtigen bzw. schwer flüchtigen Stabilisatoren werden hingegen über den Sumpfabzug der Destillationskolonne getrennt. Geeignete nicht flüchtige Stabilisatoren zur Entwässerung der Roh-Blausäure sind beispielsweise Schwefelsäure und Phosphorsäure, die anstelle von flüchtigen Stabilisatoren wie beispielsweise Schwefeldioxid im Verfahren gemäß WO 2004/092068 eingesetzt werden. WO 2004/092068 liefert jedoch keinen Hinweis darauf, dass die so aufgereinigte und folglich auch von den zugesetzten Stabilisatoren befreite Blausäure in irgendeiner Form mit Formaldehyd zur Herstellung von FACH sowie zur weiteren Umsetzung zu AAN bzw. EDA eingesetzt werden kann.

WO 2008/104552 betrifft ein Verfahren zur Herstellung eines Ethylenamingemisches, wobei ein Aminonitrilgemisch, enthaltend mindestens zwei alpha-Aminonitrile zu jeweils mindestens 5 Gew.-% in Gegenwart eines Katalysators und gegebenenfalls einem Lösungsmittel hydriert wird. Als alpha-Aminonitril kann unter anderem AAN eingesetzt werden, wodurch im Ethylenamingemisch EDA vorliegen kann. Das AAN kann wiederum aus FACH und Ammoniak hergestellt werden, das durch Umsetzung von Formaldehyd und Blausäure erhalten wird. Wie im Fall von WO 2008/104579 ist auch in WO 2008/104552 keine Angabe enthalten, dass die eingesetzte Blausäure frei von sauren Stabilisatoren, insbesondere frei von Schwefeldioxid ist.

WO 2011/067226 betrifft ein Verfahren zur Destillation eines Gemisches, enthaltend Wasser, Ethylendiamin und N-Methylethylendiamin, wobei das Gemisch in eine Destillationskolonne eingeleitet wird, die bei einem Kopfdruck von 10 mbar bis 4 bar betrieben wird. In diesem Gemisch müssen Wasser und Ethylendiamin in einem speziellen Verhältnis vorliegen. Das zur Destillation verwendete Gemisch ist wiederum ein Reaktionsaustrag, der durch Umsetzung von Formaldehyd, Blausäure, Ammoniak und Wasserstoff erhalten wird.

Die internationale Anmeldung PCT/EP2012/066808 offenbart ein Verfahren zur Herstellung von Aminen einer allgemeinen Formel (II) durch Hydrierung der entsprechenden Nitrile in Gegenwart eines Katalysators in Suspensionsfahrweise oder im Festbett bei einer speziellen Katalysatorbelastung. Sofern in der Formel (II) R¹ gleich Wasserstoff ist, handelt es sich bei dem entsprechenden Amin um EDA, das folglich durch Hydrierung von AAN erhalten wird. Das Verfahren gemäß PCT/EP2012/066808 führt jedoch vordergründig zur Herstellung von TETA und DETA aus den entsprechenden Nitrilen EDDN bzw. EDMN. Die Herstellung der Nitrile EDDN sowie EDMN kann wiederum aus Formaldehyd und Blausäure mit der Zwischenstufe FACH erfolgen. Der dabei eingesetzten Blausäure wird in der Regel ein saurer Stabilisator, beispielsweise Schwefeldioxid, Schwefelsäure, Phosphorsäure oder eine organische Säure wie Essigsäure zugesetzt, um die autokatalytische Polymerisation von Blausäure, die in Rohrleitungen zu Verstopfungen führen kann, zu verhindern.

Die internationale Anmeldung PCT/EP2012/066833 offenbart ein weiteres Verfahren zur Herstellung von TETA und/oder DETA durch Umsetzung von EDDN und/oder EDMN mit Wasserstoff in Gegenwart eines Katalysators. Als Katalysator wird ein Katalysator vom Raney-Typ eingesetzt und der Druck bei der Hydrierung liegt im Bereich von 170 bis 240 bar. In diesem Verfahren werden jedoch keine sonstigen Nitrile wie AAN einer Hydrierung unterzogen.

Die der vorliegenden Erfindung zugrunde liegende Aufgabe besteht somit in der Bereitstellung eines neuen Verfahrens zur Herstellung von Ethylendiamin (EDA). Gelöst wird die Aufgabe durch ein Verfahren zur Herstellung von Ethylendiamin (EDA), umfassend die Schritte a) bis c):
a) Umsetzung von Formaldehyd und Blausäure (HCN) zu Formaldehydcyanhydrin (FACH), wobei der Schwefeldioxid-Gehalt in der eingesetzten Blausäure weniger als 100 Gew.-ppm, bezogen auf die Menge an eingesetzter Blausäure, beträgt,
b) Umsetzung von FACH mit Ammoniak (NH₃) zu Aminoacetonitril (AAN),
c) Hydrierung von AAN in Gegenwart eines Katalysators unter Erhalt von EDA.

Ein wesentlicher Vorteil des erfindungsgemäßen Verfahrens ist darin zu sehen, dass die Standzeit des Hydrierkatalysators (also des Katalysators, der in Schritt c) eingesetzt wird) verbessert wird. Dieser Effekt tritt insbesondere dann ein, wenn die zur FACH-Herstellung gemäß Schritt a) eingesetzte Blausäure vollständig oder zumindest weitgehend frei von Schwefeldioxid sowie gegebenenfalls von weiteren sauren Stabilisatoren wie Schwefelsäure oder Phosphorsäure ist.

Die Verwendung von (weitgehend) SO₂-freier Blausäure in Schritt a) wirkt sich somit insbesondere positiv auf das Leistungsvermögen des in Schritt c) eingesetzten Hydrierkatalysators aus. Die AAN-Hydrierung kann somit über lange Zeiten mit hohen EDA-Ausbeuten ohne wesentliches Absinken der Katalysator-Aktivität durchgeführt werden. Insbesondere SO₂ neigt dazu, unter den bei einer Nitrilhydrierung gängigen Verfahrensbedingungen in Gegenwart des Katalysators zu disproportionieren, wobei unter anderem Sulfide entstehen, die wesentlich zur Verminderung des Leistungsvermögens eines Hydrierkatalysators beitragen. Eine solche Disproportionierung wird hingegen bei der Verwendung von Schwefelsäure antelle von SO₂ nicht oder nur deutlich verringert festgestellt.

Darüber hinaus werden durch die Verwendung von (weitgehend) SO₂-freier Blausäure in Schritt a) auch die Ausbeuten der Teilschritte positiv beeinflusst, was sich wiederum auch auf die EDA-Gesamtausbeute auswirkt.

Ein weiterer Vorteil des erfindungsgemäßen Verfahrens ist in der Flexibilität zu sehen. Das erfindungsgemäße Verfahren kann diskontinuierlich, halbkontinuierlich sowie insbesondere kontinuierlich zur Herstellung von EDA aus Formaldehyd, Blausäure sowie Ammoniak und Wasserstoff eingesetzt werden. Weiterhin sind die optional durchzuführenden Aufarbeitungsschritte des erfindungsgemäßen Verfahrens in apparativer und energetischer Sicht vorteilhaft.

Weiterhin wirkt es sich im erfindungsgemäßen Verfahren vorteilhaft aus, wenn in Schritt a) die Blausäure in einem leichten Unterschuss gegenüber Formaldehyd eingesetzt wird und/oder in Schritt b) Ammoniak mit einem hohen molaren Überschuss gegenüber dem in Schritt a) hergestellten FACH eingesetzt wird. Weiterhin ist es vorteilhaft, wenn in Schritt b) der Druck so gewählt wird, dass er leicht höher ist als in der nachfolgenden Hydrierung gemäß Schritt c), da auf diese Weise ein Förderorgan (z. B. eine Pumpe) entfallen kann.

Ein weiterer Vorteil kann dann erzielt werden, sofern der in Schritt b) eingesetzte überschüssige bzw. nicht umgesetzte Ammoniak im Anschluss an die Hydrierung gemäß Schritt c) aus dem Reaktionsgemisch (Hydrierungsprodukt) abgetrennt und rückgeführt wird. Vorteilhafterweise wird die Ammoniakabtrennung in zwei Stufen durchgeführt, wobei durch entsprechende Druck- sowie Temperaturregelung die Ammoniakabtrennung bzw. Ammoniakrückgewinnung effizient betrieben werden kann, beispielsweise in Form einer geschlossenen Kondensation.

Im Rahmen der vorliegenden Erfindung beträgt der Schwefeldioxid-Gehalt in der eingesetzten Blausäure weniger als 100 Gew.-ppm, bevorzugt weniger als 50 Gew.-ppm und insbesondere weniger als 30 Gew.-ppm. Die vorstehenden Angaben beziehen sich auf die Menge an eingesetzter Blausäure.

Weiterhin wird im Rahmen der vorliegenden Erfindung unter dem Begriff "vollständig frei von Schwefeldioxid" Folgendes verstanden: Der Schwefeldioxid-Gehalt in der eingesetzten Blausäure beträgt weniger als 10 Gew.-ppm, bevorzugt weniger als 1 Gew.-ppm, insbesondere weniger als 0,1 Gew.-ppm. Die vorstehenden Angaben beziehen sich auf die Menge an eingesetzter Blausäure.

Sinngemäße Aussagen hinsichtlich des Gehaltes an sonstigen Stabilisatoren gelten im Rahmen der vorliegenden Erfindung auch für die Ausführungen, in denen die Blausäure vollständig bzw. weitgehend frei von sonstigen sauren Stabilisatoren, wie Schwefelsäure, Phosphorsäure oder Essigsäure, ist.

Nachfolgend wird die vorliegende Erfindung weiter präzisiert.

Im erfindungsgemäßen Verfahren erfolgt in Schritt a) die Umsetzung von Formaldehyd und Blausäure (HCN) zu Formaldehydcyanhydrin (FACH), wobei die Blausäure vollständig frei oder weitgehend frei von Schwefeldioxid (SO₂) ist.

Formaldehyd ist eine im Handel allgemein erhältliche Chemikalie. Vorzugsweise wird Formaldehyd in Form einer wässrigen Lösung eingesetzt. Bevorzugt handelt es sich dabei um wässrigen Formaldehyd mit einem Formaldehydanteil von 20 bis 60 Gew.-% [Mol/Mol], besonders bevorzugt mit einem Formaldehydanteil von 25 bis 55 Gew.-%.

Blausäure ist ebenfalls eine im Handel allgemein erhältliche Chemikalie. Blausäure kann großtechnisch nach im Wesentlichen drei verschiedenen Verfahren hergestellt werden. Nach einem ersten Verfahren kann Blausäure durch Ammoxidation von Methan mit Sauerstoff und Ammoniak (Andrussow-Verfahren) erhalten werden. Nach einem zweiten Verfahren kann Blausäure aus Methan und Ammoniak durch Ammondehydrierung in Abwesenheit von Sauerstoff erhalten werden. Schließlich kann Blausäure großtechnisch durch Dehydratisierung von Formamid hergestellt werden.

Blausäure kann flüssig oder gasförmig, in reiner Form oder als wässrige Lösung eingesetzt werden. Bevorzugt wird Blausäure als 50 bis 100 gew.-%ige, besonders bevorzugt als 75 bis 100 gew.-%ige wässrige Lösung eingesetzt. Blausäure wird vorzugsweise in einer Reinheit von 90 Gew.-% oder mehr eingesetzt.

Wie vorstehend bereits ausgeführt (inklusive der konkreten Zahlenwerte), kann im Rahmen des erfindungsgemäßen Verfahrens die Blausäure vollständig frei oder weitgehend frei von Schwefeldioxid (SO₂) sein. Schwefeldioxid kann als Stabilisator der Blausäure (beispielsweise nach deren Herstellung) direkt zugegeben werden. Dem Fachmann ist bekannt, dass sich in Gegenwart von Wasser mit SO₂ schwefelige Säure (H₂SO₃) bildet. Messmethoden zur Bestimmung des SO₂-Gehaltes von Blausäure sind dem Fachmann bekannt, beispielsweise kann dies durch Ionenchromatographie erfolgen.

Da in der kommerziell erhältlichen Blausäure in aller Regel Stabilisatoren, insbesondere saure Stabilisatoren, enthalten sind, wird im Rahmen der vorliegenden Erfindung entweder frisch synthetisierte und somit stabilisatorfreie Blausäure eingesetzt oder die vorhandenen Stabilisatoren, insbesondere Schwefeldioxid, werden nach dem Fachmann bekannten Methoden direkt vor dem Einsatz in Schritt a) entfernt. Stabilisatorfreie Blausäure, ausgehend von nicht-flüchtige Stabilisatoren, insbesondere Schwefelsäure oder Phosphorsäure, enthaltender Blausäure, lässt sich nach den in WO 2004/092068 beschriebenen Methoden destillativ herstellen. Dabei wird die Blausäure über Kopf abdestilliert, die entsprechenden Stabilisatoren fallen als Sumpfprodukte an.

Stabilisatorfreie Blausäure, ausgehend von flüchtige Stabilisatoren, insbesondere von Schwefeldioxid, enthaltender Blausäure, kann gemäß US-A 2 571 099 infolge von Durchleiten eines Inertgases durch die Blausäure erhalten werden.

In einer bevorzugten Ausführungsform der vorliegenden Erfindung wird in Schritt a) Blausäure eingesetzt, die vollständig frei von Stabilisatoren ist. Die entsprechenden Zahlenwerte für den Begriff "vollständig frei" wurden vorstehend bereits definiert. Als Stabilisatoren werden in diesem Zusammenhang insbesondere Schwefeldioxid, Schwefelsäure, Phosphorsäure, Essigsäure, Oxalsäure sowie gegebenenfalls sonstige Säuren angesehen, die dem Fachmann als saure Stabilisatoren für Blausäure bekannt sind.
Die Umsetzung von Formaldehyd, vorzugsweise wässrigem Formaldehyd, und Blausäure zu FACH kann diskontinuierlich, halbkontinuierlich oder kontinuierlich durchgeführt werden. Vorzugsweise erfolgt sie in einem rückvermischten Reaktor mit Wärmeabfuhr, beispielsweise unter Verwendung eines Wärmetauschers. Als Reaktoren zur Durchführung von Schritt a) können insbesondere Rührreaktoren, Schlaufenreaktoren oder Rohrreaktoren verwendet werden.

Schritt a) kann prinzipiell bei beliebigen Temperaturen durchgeführt werden, vorzugsweise beträgt die Reaktionstemperatur 0 bis 70 °C, mehr bevorzugt 10 bis 50 °C, besonders bevorzugt 20 bis 45 °C.

Der Druck in Schritt a) wird dabei so gewählt, dass das Reaktionsgemisch flüssig vorliegt.

Vorzugsweise wird die Blausäure gegenüber Formaldehyd äquimolar oder in einem leichten Unterschuss gefahren. Mehr bevorzugt beträgt das Molverhältnis von HCN zu Formaldehyd 0,85 bis 1,0 zu 1 [Mol/Mol], noch mehr bevorzugt 0,9 bis 1,0 zu 1 [Mol/Mol], insbesondere 0,95 bis 1,0 zu 1 [Mol/Mol].

Weiterhin ist es bevorzugt, dass das Reaktionsgemisch mit Hilfe einer Base, vorzugsweise mit Natronlauge, auf einen pH-Wert von 3,5 bis 6,5, bevorzugt 4,0 bis 6,0, besonders bevorzugt von 5,5 eingestellt wird.

Die Verweilzeit in der FACH-Synthese beträgt 1 Minute bis 1 Stunde, bevorzugt 5 Minuten bis 30 Minuten.

Der HCN-Umsatz in der FACH-Synthese beträgt > 99 % (bestimmt durch Titration nach Volhard), die FACH-Ausbeute beträgt > 98 % (bestimmt durch kombinierte Titration nach Volhard und Liebig).

Falls die erhaltene, vorzugsweise wässrige, FACH-Lösung beispielsweise auf eine 50 bis 80 gew.-%ige Lösung aufkonzentriert werden soll, ist es vorteilhaft, den pH-Wert im Reaktionsgemisch von Schritt a) auf Werte < 5,5, bevorzugt < 3,5, abzusenken. Dies kann beispielsweise durch Zugabe von Mineralsäuren wie Schwefelsäure oder Phosphorsäure erreicht werden.

Im erfindungsgemäßen Verfahren erfolgt in Schritt b) die Umsetzung von FACH mit Ammoniak (NH₃) zu Aminoacetonitril (AAN).

Erfindungsgemäß setzt man im Allgemeinen den Reaktionsaustrag aus Schritt a), also das FACH bzw. ein FACH enthaltendes Gemisch, ohne Aufarbeitung und ohne die zusätzliche Verwendung eines Lösungsmittels mit Ammoniak zu AAN um. Ammoniak kann in Schritt b) nicht nur als Edukt bei der AAN-Herstellung, sondern auch als Lösungsmittel verwendet werden. Gegebenenfalls kann eine in Schritt a) erhaltene wässrige FACH-Lösung durch Abdampfen von Wasser aufkonzentriert werden.

Die Umsetzung von FACH mit Ammoniak kann diskontinuierlich, halbkontinuierlich oder kontinuierlich erfolgen. Die Umsetzung kann in jeder geeigneten und dem Fachmann bekannten Vorrichtung erfolgen. Vorzugsweise wird in Schritt b) ein adiabates oder ein gekühltes Strömungsrohr ohne Rückvermischung oder ein Reaktor mit Kolbenströmungscharakteristik eingesetzt. Auf diese Weise kann die Bildung von störenden Nebenkomponenten aus FACH und AAN, erkennbar an der Farbe, vermieden werden.

In Schritt b) beträgt die Temperatur in der Regel 0 bis 150 °C, bevorzugt 50 bis 100 °C, besonders bevorzugt 70 bis 90 °C.

In Schritt b) kann der Druck prinzipiell beliebig eingestellt werden. Vorzugsweise beträgt in Schritt b) der Druck 20 bis 400 bar, insbesondere 80 bis 270 bar. Vorzugsweise ist in Schritt b) der Druck so hoch ist, dass das Reaktionsgemisch flüssig ist. Weiterhin vorzugsweise ist der Druck in Schritt b) höher als in Schritt c). Beispielsweise kann der Druck in Schritt b) um 5 bis 20 bar höher sein als in Schritt c).

Das Molverhältnis zwischen FACH und Ammoniak kann prinzipiell beliebig sein, in der Regel wird jedoch zumindest eine äquimolare Menge an Ammoniak eingesetzt, vorzugsweise wird Ammoniak in hohem molarem Überschuss zu FACH gefahren. Vorzugsweise beträgt in Schritt b) das Molverhältnis von FACH zu Ammoniak 1 : 2 bis 1 : 15 [Mol/Mol], mehr bevorzugt 1 : 5 bis 1 : 30 [Mol/Mol], insbesondere 1 : 10 bis 1 : 20 [Mol/Mol].

Die Verweilzeit des Reaktionsgemisches in der entsprechenden Vorrichtung beträgt vorzugsweise 0,1 bis 20 Minuten, besonders bevorzugt 1,0 bis 10 Minuten.

Die AAN-Ausbeute beträgt (bezogen auf FACH) vorzugsweise ≥ 95 %. Weiterhin ist das Gewichtsverhältnis von AAN zu IDAN vorzugsweise ≥ 99 : 1.

In Schritt b) enthält der Reaktionsaustrag vorzugsweise 10 bis 50 Gew.-% AAN, 10 bis 80 Gew.-% Ammoniak, < 1 Gew.-% FACH, < 1 Gew.-% IDAN. Der Rest ist Wasser, das in Schritt b) bei der Herstellung von AAN gebildet wird bzw. bereits bei der FACH-Herstellung, gemeinsam mit den Edukten, eingesetzt wurde.

In einer bevorzugten Ausführungsform der vorliegenden Erfindung wird das in Schritt b) erhaltene AAN
i) ohne destillative Aufarbeitung und/oder ohne Wasserabtrennung als Roh-AAN in Schritt c) hydriert, oder
ii) einer adsorptiven Reinigung unter Verwendung eines Ionentauschers oder eines Metalloxids unterzogen, bevor es gemäß Schritt c) hydriert wird.

Im erfindungsgemäßen Verfahren wird in Schritt c) die Hydrierung von AAN in Gegenwart eines Katalysators unter Erhalt von Ethylendiamin (EDA) durchgeführt.

Die Hydrierung unter Erhalt von EDA erfolgt im Allgemeinen durch Umsetzung von AAN mit Wasserstoff in Gegenwart des Katalysators ("Hydrierkatalysator"). Dabei werden mindestens zwei Mol Wasserstoff pro Mol AAN benötigt. Das in Schritt b) erhaltene AAN kann direkt der Hydrierung gemäß Schritt c) unterzogen werden, gegebenenfalls können aber noch Aufarbeitungsschritte zwischen Schritt b) und Schritt c) durchgeführt werden, wie weiter unten näher erläutert.

Der Wasserstoff kommt im Allgemeinen technisch rein zum Einsatz. Der Wasserstoff kann auch in Form eines Wasserstoff enthaltenden Gases, das heißt mit Beimengungen anderer Inertgase, wie Stickstoff, Helium, Neon, Argon oder Kohlendioxid, zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO, enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

Als Katalysatoren können prinzipiell alle dem Fachmann für eine Nitrilhydrierung bekannten Katalysatoren eingesetzt werden. Als Katalysatoren zur Hydrierung (Hydrierkatalysatoren) der Nitrilfunktion von AAN können somit beispielsweise Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten.

Darin eingeschlossen sind so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten.

In einer besonders bevorzugten Ausführungsform werden bei Hydrierung von AAN Raney-Katalysatoren eingesetzt, bevorzugt Raney-Kobalt- oder Raney-NickelKatalysatoren und besonders bevorzugt ein Raney-Kobalt-Katalysator, der als Promotor mindestens eines der Elemente Ni, Cr oder Fe enthält. Der Raney-Kobalt-Katalysator ist also mit mindestens einem dieser Elemente dotiert. Erfindungsgemäß werden die Raney-Katalysatoren bevorzugt als suspendierte Raney-Katalysatoren eingesetzt.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die zur Hydrierung eingesetzten Katalysatoren können vor dem Einsatz beispielsweise außerhalb eines Reaktors oder im Reaktor nach dem Fachmann bekannten Methoden aktiviert werden. Eine Sonderstellung bei der Aktivierung haben die Skelett-Katalysatoren, die durch Laugung mit wässriger Base, wie z. B. in EP-A 1 209 146 beschrieben, aktiviert werden können. Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Kobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Kobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrO₂,1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O3 bzw. MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält. Beispielsweise der in dieser Schrift konkret offenbarte Katalysator mit der Zusammensetzung 31 ,5 Gew.-% ZrO₂, 50 Gew.-% NiO, 17 Gew.-% CuO und 1 ,5 Gew.-% MoO₃. Ebenso geeignet sind die in WO-A-99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als "Metallschwamm" aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach zum Beispiel mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw. Die Aktivierung der Katalysatoren durch Auslaugen der löslichen Komponente (typscherweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

In einer bevorzugten Ausführungsform wird erfindungsgemäß ein Raney-Kobalt-Skelett-Katalysator eingesetzt, der aus einer Co/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Ni oder Cr enthält.

Solche Katalysatoren enthalten typischerweise neben Kobalt noch 1 - 30 Gew.-% Al, besonders 2 - 12 Gew.-% Al, ganz besonders 3 - 6 Gew.-% Al, 0 - 10 Gew.-% Cr, bei-sonders 0,1 - 7 Gew.-% Cr, ganz besonders 0,5 - 5 Gew.-% Cr, insbesondere 1,5 - 3,5 Gew.-% Cr, 0 - 10 Gew.-% Fe, besonders 0,1 - 3 Gew.-% Fe, ganz besonders 0,2 - 1 Gew.-% Fe, und/oder 0 - 10 Gew.-% Ni, besonders 0,1 - 7 Gew.-% Ni, ganz besonders 0,5 - 5 Gew. % Ni, insbesondere 1 - 4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf: Al: 2-6 Gew.-%, Co: ≥ 86 Gew.-%, Fe: 0-1 Gew.-%, Ni: 1-4 Gew.-%, Cr: 1,5-3,5 Gew.-%.

Ebenfalls kann erfindungsgemäß ein Nickel-Skelett-Katalysator eingesetzt werden, der aus einer Ni/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Cr enthält.

Solche Katalysatoren enthalten typischerweise neben Nickel noch 1 - 30 Gew.-% Al, besonders 2 - 20 Gew.-% Al, ganz besonders 5 - 14 Gew.-% Al, 0 - 10 Gew.-% Cr, besonders 0,1 - 7 Gew.-% Cr, ganz besonders 1 - 4 Gew.-% Cr, und/oder 0 - 10 Gew.-% Fe, besonders 0,1 - 7 Gew.-% Fe, ganz besonders 1 - 4 Gew.-% Fe, wobei die Gewichtsangaben jeweils auf das Katalysatorgesamtgewicht bezogen sind.

Als Katalysator im erfindungsgemäßen Verfahren kann zum Beispiel vorteilhaft ein Nickel-Skelett-Katalysator A 4000 der Firma Johnson Matthey eingesetzt werden. Dieser Katalysator weist folgende Zusammensetzung auf:
Al: <14 Gew.-%, Ni: ≥ 80 Gew.-%, Fe: 1-4 Gew.-%, Cr: 1-4 Gew.-%.

Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierliche entnommen, ex situ regeneriert und zurückgeführt.

In Schritt c) ist es bevorzugt, dass die Katalysatorbelastung 0,1 bis 3 Gramm AAN pro Gramm Katalysator und Stunde, vorzugsweise 0,2 bis 1,5 Gramm AAN pro Gramm Katalysator und Stunde, insbesondere 0,4 bis 1,2 Gramm AAN pro Gramm Katalysator und Stunde beträgt.

Weiterhin ist es in Schritt c) bevorzugt, dass der Katalysator eine BET-Oberfläche von 10 bis 100 m2 pro Gramm Katalysator, vorzugsweise von 20 bis 500 m² pro Gramm Katalysator, insbesondere von 30 bis 100 m² pro Gramm Katalysator, aufweist. Die Messung zur Bestimmung der BET-Oberfläche ist dem Fachmann bekannt, in der Regel wird hierzu die BET-Methode gemäß DIN 66131 angewandt.

Die Hydrierung kann prinzipiell bei beliebigen Temperaturen durchgeführt werden, vorzugsweise erfolgt die Hydrierung bei einer Temperatur von 20 bis 150 °C, mehr bevorzugt bei 40 bis 120 °C, besonders bevorzugt bei 70 bis 110 °C.

Prinzipiell kann bei der Hydrierung jeder beliebige Druck angewandt werden, vorzugsweise beträgt bei der Hydrierung der Druck 40 bis 400 bar, mehr bevorzugt 80 bis 300 bar, besonders bevorzugt 100 bis 270 bar.

Vorzugsweise wird das AAN mit einer Rate der Hydrierung zugeführt, die nicht größer ist als die Rate, mit der das AAN mit Wasserstoff bei der Hydrierung reagiert. Die Zuführrate ist bevorzugt so einzustellen, dass quasi Vollumsatz erreicht wird. Dieses wird durch Druck, Temperatur, Menge und Art des Katalysators, Menge des AAN im zugeführten Gemisch, die Durchmischungsgüte des Reaktorinhaltes sowie die Verweilzeit etc. beeinflusst.

Die Umsetzung von AAN mit Wasserstoff in Gegenwart von Katalysatoren kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des AANs und des Katalysators mit dem Wasserstoff unter Druck möglich ist.

Die Hydrierung in Suspensionsfahrweise kann in einem Rührreaktor, Strahlschlaufenreaktor, Strahldüsenreaktor, Blasensäulenreaktor bzw. in einer Kaskade derartiger gleicher oder verschiedener Reaktoren durchgeführt werden.

Bevorzugt setzt man Strahlschlaufenreaktoren mit einem externen Kreislauf ein, an dem durch Kühlung die Reaktionswärme entzogen werden kann.

Unter Stahlschlaufenreaktor versteht man einen mit einer Düse versehenen Reaktor, an der ein Strahl erzeugt wird, der gasförmigen Wasserstoff in die Flüssigkeit einträgt. Die Düse wird mit den Eduktströmen, bevorzugt mit einem extern geführten Flüssigkeitskreislauf, besonders bevorzugt mit Edukten und dem extern geführten Flüssigkeitskreislauf betrieben. In einer bevorzugten Variante ist im Reaktor ein zusätzliches Rohr enthalten, der einen Impulsaustausch verhindert, so dass sich ein interner Kreislauf ausbilden kann.

Die Hydrierung an einem Festbettkatalysator findet bevorzugt in einem oder mehreren Rohrreaktoren aber auch Rohrbündelreaktoren statt.

Der Katalysator kann suspendiert oder in Form von Stranglagen oder Tabletten vorliegen. Bevorzugt wird ein suspendierter Katalysator verwendet, der durch Filtration mit Filterkerzen oder Querstromfiltration im Reaktor zurückgehalten wird. Besonders bevorzugt wird dabei die Questromfiltration im externen Kreislaufstrom eines Strahlschlaufenreaktors realisiert.

Die Hydrierung der Nitrilgruppen findet unter Freisetzung von Wärme statt, die in der Regel abgeführt werden muss. Die Wärmeabfuhr kann durch eingebaute Wärmeüberträgerflächen, Kühlmäntel oder außen liegende Wärmeüberträger in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes.

Insbesondere kann der Kreislaufstrom mittels eines externen Wärmeüberträgers auf einfache und kostengünstige Weise gekühlt und somit die Reaktionswärme abgeführt werden.

Der Reaktor kann auch adiabat betrieben werden. Bei adiabatem Betrieb des Reaktors kann der Temperaturanstieg im Reaktionsgemisch durch Abkühlung der Zuläufe oder durch Zufuhr von "kaltem" organischem Lösungsmittel begrenzt werden. Alternativ kann man eine Kühlung duch gezieltes Zulassen einer Verdampfung/Verdunstung des Lösungsmittels innerhalb des Reaktors zulassen.

Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbett- einem Suspensionsreaktor nachgeschaltet.

Der Katalysator kann in einem Festbett angeordnet (Festbettfahrweise) sein oder im Reaktionsgemisch suspendiert (Suspensionsfahrweise) sein.

In einer besonders bevorzugten Ausführungsform ist der Katalysator im zu hydrierenden Reaktionsgemisch suspendiert.

Die Absetzgeschwindigkeit des Hydrierkatalysators in dem gewählten Lösungsmittel sollte niedrig sein, damit der Katalysator gut in Suspension gehalten werden kann.

Die Partikelgröße der eingesetzten Katalysatoren beträgt bei der Suspensionsfahrweise daher bevorzugt zwischen 0,1 und 500 µm, insbesondere 1 und 100 µm.

Wird die Hydrierung von AAN in Suspensionsfahrweise kontinuierlich durchgeführt, so wird AAN bevorzugt kontinuierlich dem Reaktor zugeführt und aus dem Reaktor kontinuierlich ein Strom entfernt, der das Hydrierungsprodukt EDA enthält.

Die Menge an Katalysator bei der diskontinuierlichen Suspensionsfahrweise beträgt bevorzugt 1 bis 60 Gew.-%, besonders bevorzugt 5 bis 40 Gew.-%, und ganz besonders bevorzugt 20 bis 35 Gew.-%, bezogen auf das gesamte Reaktionsgemisch.

Die Katalysatorkonzentration in einem kontinuierlich betriebenen Reaktor beträgt 1 bis 60 %, bevorzugt 5 bis 40 %, besonders bevorzugt 20 bis 35 Gew%.

Die Verweilzeit im Reaktor beträgt bei diskontinuierlicher Suspensions-Fahrweise bevorzugt 0,1 bis 6 Stunden, besonders bevorzugt 0,5 bis 2 Stunden.

Die Verweilzeit im Reaktor beträgt bei kontinuierlicher Suspensionsfahrweise bevorzugt 0,1 bis 6 Stunden, besonders bevorzugt 0,5 bis 2 Stunden.

Wird die Reaktion in Suspensionsfahrweise in einem Rührreaktor durchgeführt, so liegt der Leistungseintrag über den Rührer bevorzugt bei 0,1 bis 100 KW pro m³, bevorzugt 1 bis 10 kW/m³.

Gebrauchter Katalysator kann durch Filtration, Zentrifugieren oder Querstromfiltration abgetrennt werden. Dabei kann es notwendig sein, Verluste an ursprünglicher Katalysatormenge durch Abrieb und/oder Deaktivierung durch Zugabe von frischem Katalysator auszugleichen.

Im Anschluss an die Hydrierung kann der Austrag aus der Hydrierung gegebenenfalls weiter aufgereinigt werden. Der Katalysator kann nach dem Fachmann bekannten Methoden abgetrennt werden. In der Regel wird nach Abtrennung des Katalysators der während der Hydrierung vorhandene Wasserstoff abgetrennt.

Die Abtrennung von Wasserstoff erfolgt bevorzugt durch Absenkung des Drucks, bei dem die Hydrierung durchgeführt wurde, auf einen Wert, bei dem Wasserstoff gasförmig ist, die anderen Komponenten im Reaktionsaustrag aber in der Flüssigphase vorliegen. Vorzugsweise wird der Reaktionsaustrag von einem Hydrierdruck von bevorzugt 60 bis 325 bar, besonders bevorzugt 100 bis 280 bar, und ganz besonders bevorzugt 170 bis 240 bar auf einen Druck von 5 bis 50 bar in einen Behälter entspannt. Am Kopf des Behälters werden Wasserstoff, gegebenenfalls Ammoniak, sowie gegebenenfalls geringe Menge an verdampften Leichtsiedern oder Lösungsmitteln, erhalten. Wasserstoff und gegebenenfalls Ammoniak können in die Hydrierung von AAN zurückgeführt werden.

Der Hydrieraustrag enthält zunächst das Zielprodukt EDA. Gegebenenfalls kann im Hydrieraustrag auch nicht umgesetztes Edukt in Form von AAN bzw. dessen Vorstufen enthalten sein. Vorzugsweise ist im Hydrieraustrag kein AAN mehr enthalten. Im Allgemeinen enthält der Hydrieraustrag neben EDA auch Ammoniak und Wasser, die in den vorausgegangenen Verfahrensstufen beispielsweise als Edukt oder Lösungsmittel eingesetzt wurden und/oder entstanden sind. Bei der Hydrierung von AAN entsteht in aller Regel als Nebenprodukt Diethylentriamin (DETA) sowie gegebenenfalls N-Methylethylendiamin (N-MEDA). Erfindungsgemäß enthält der Hydrieraustrag neben EDA 1 bis 10 % DETA und 1 bis 5 % N-MEDA bezogen auf das lösungsmittelfreie EDA.

Die Ausbeute an Zielprodukt EDA beträgt vorzugsweise 85 bis 100 %, bevorzugt 90 bis 100%.

Vorzugsweise erfolgt nach der Hydrierung gemäß Schritt c) des erfindungsgemäßen Verfahrens die Abtrennung von EDA aus dem Hydrierungsprodukt. Bevorzugt erfolgt die Abtrennung von EDA destillativ nach dem Fachmann bekannten Methoden. Weiterhin ist es bevorzugt, dass die EDA-Abtrennung im Anschluss an die weiter unten beschriebene Ammoniakabtrennung gemäß Schritt d) und/oder die Wasserabtrennung durchgeführt wird. Besonders bevorzugt erfolgt eine destillative EDA-Abtrennung erst nachdem zunächst die Ammoniak-Abtrennung gemäß Schritt d) und anschließend die Wasserabtrennung durchgeführt wurde.

In einer bevorzugten Ausführungsform der vorliegenden Erfindung wird im Anschluss an die Hydrierung gemäß Schritt c) als Schritt d) eine Ammoniakabtrennung aus dem Hydrierungsprodukt durchgeführt. Vorzugsweise erfolgt die Ammoniakabtrennung aus dem Hydrierungsprodukt, bevor eine Wasserabtrennung sowie eine destillative Abtrennung von EDA durchgeführt wird. Besonders bevorzugt wird das gemäß Schritt d) abgetrennte Ammoniak nach Schritt b) rückgeführt. Vorzugsweise wird das rückgeführte Ammoniak mit frischem Ammoniak vermischt und anschließend gemeinsam in die Vorrichtung zur Durchführung von Schritt b) eingespeist.

Weiterhin ist es bevorzugt, dass Schritt d) in zwei Stufen durchgeführt wird, wobei in der ersten Stufe aus einer Kolonne (K1) Ammoniak über Kopf bei 20 bis 70 °C abgezogen und auskondensiert wird, die Sumpftemperatur kleiner als 220 °C beträgt und aus dem Sumpf ein NH₃-abgereicherter Strom in eine zweite Kolonne (K2) überführt wird, und wobei in der zweiten Stufe in der zweiten Kolonne (K2) EDA aus dem Sumpf abgetrennt wird, wobei das EDA (weitgehend) frei von Ammoniak ist, und gegebenenfalls ein NH₃-haltiger Strom in die Kolonne (K1) rückgeführt wird.

Als Kolonnen (K1) und (K2) können alle dem Fachmann hierfür bekannten Destillationsvorrichtungen verwendet werden. In Kolonne (K1) beträgt die Temperatur beim Kolonnenkopf ("Kondensationstemperatur") vorzugsweise 20 bis 70 °C, insbesondere 35 bis 60 °C. Vorzugsweise wird die Temperatur in der Kolonne (K1), insbesondere im Kopfbereich, über den Druck geregelt. Der Druck kann anhand von dem Fachmann bekannten Dampfdrucktabellen von Ammoniak bestimmt werden. Die Sumpftemperatur der Kolonne (K1) ist vorzugsweise < 200 °C, besonders bevorzugt < 190 °C. Die Sumpftemperatur wird vorzugsweise über den Ammoniakgehalt eingestellt, der über Sumpf aus der Kolonne (K1) abgezogen wird. In der Regel wird eine Sumpftemperatur von 140 °C nicht unterschritten.

In der zweiten Stufe wird der Kolonnendruck vorzugsweise so eingestellt, dass der Sumpfaustrag der Kolonne (K2) ammoniakfrei bzw. weitgehend frei von Ammoniak ist. Weitgehend frei von Ammoniak ist der Sumpfaustrag dann, wenn der Ammoniakgehalt < 1 Gew.-%, vorzugsweise < 0,5 Gew.-% ist, ammoniakfrei ist der Sumpfaustrag dann, wenn der Ammoniakgehalt < 0,1 Gew.-%, vorzugsweise 0,01 Gew.-% ist.

Die Sumpftemperaturen in der Kolonne (K2) entsprechen den Sumpftemperaturen, die vorstehend im Zusammenhang mit der Kolonne (K1) aufgeführt worden sind. Aus dem oberen Teil der Kolonne (K2), vorzugsweise über den Kolonnenkopf, wird Ammoniak abgetrennt. Vorzugsweise enthält dieser Strom neben Ammoniak auch Wasser. Dabei wird der Wassergehalt vorzugsweise so eingestellt, dass die Kopftemperatur der Kolonne (K2) bzw. die Kondensationstemperatur den vorstehend für die Kolonne (K1) angegebenen Temperaturbereichen am Kolonnenkopf entspricht. Vorzugsweise wird der NH₃-haltige Strom, der vorzugsweise aus dem Kolonnenkopf von K2 abgezogen wird und der zusätzlich Wasser enthält, in die Kolonne (K1) zur ersten Stufe der Ammoniakabtrennung rückgeleitet. Besonders bevorzugt führt man den Kondensator in Form einer geschlossenen Kondensation aus. Dies kann durch Rückvermischung des Kondensates (Einführung eines Kreislaufes über den Kondensator) oder durch Kondensation im Gleichstrom ausgeführt werden.

In einer weiteren Ausführungsform der vorliegenden Erfindung wird nach der Hydrierung gemäß Schritt c) Wasser aus dem Hydrierungsprodukt abgetrennt, vorzugsweise erfolgt die Wasserabtrennung im Anschluss an die Ammoniakabtrennung gemäß Schritt d).

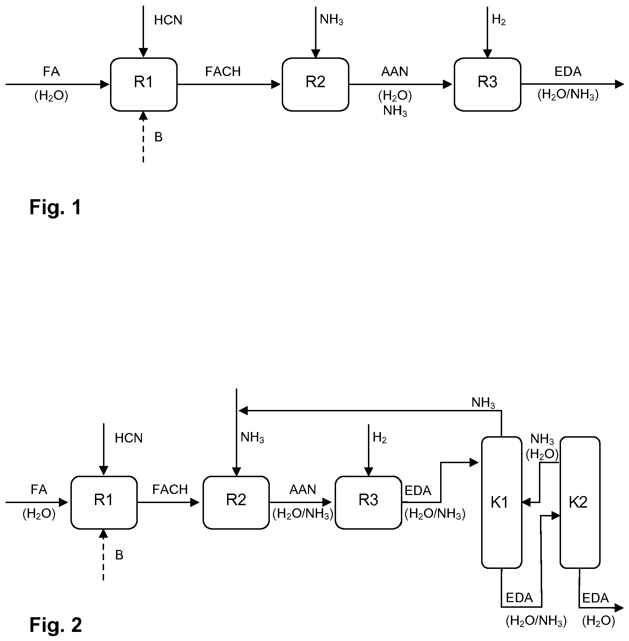

In Figur 1 wird das erfindungsgemäße Verfahren in seiner Grundform nochmals verdeutlicht. "FA" bedeutet Formaldehyd, "B" bedeutet Base. Die Verwendung einer Base ist hier nur optional, was durch die gestrichelte Linie angedeutet ist. Die Verfahrensschritte a) bis c) werden vorzugsweise in entsprechenden Reaktoren durchgeführt, die in Figur 1 sinngemäß als "R1" bis "R3" dargestellt sind. Die Hauptkomponenten (Edukte oder Produkte) der jeweiligen Schritte sind unter Verwendung von Pfeilen entsprechend aufgeführt, in den Klammern sind die wichtigsten Nebenprodukte bzw. nicht umgesetzten Edukte der einzelnen Schritte angegeben. Etwaige weitere Aufarbeitungsschritte oder spezielle Ausgestaltungen der jeweiligen Vorrichtungen, beispielsweise die Verwendung von zwei in Reihe geschalteten Hydrierreaktoren für Schritt c), sind in Figur 1 nicht schematisch dargestellt, sie können jedoch gegebenenfalls zusätzlich durchgeführt werden bzw. enthalten sein.

Eine bevorzugte Ausführungsform der vorliegenden Erfindung wird in Figur 2 zusätzlich verdeutlicht. In Figur 2 haben die Abkürzungen, Pfeile und sonstigen Symbole eine sinngemäße Bedeutung wie vorstehend für Figur 1 ausgeführt. Gegenüber der Ausführungsform gemäß Figur 1 wird in der Ausführungsform gemäß Figur 2 zusätzlich eine Abtrennung sowie Rückführung von Ammoniak (gemäß Schritt d)) durchgeführt. Die Ammoniakabtrennung kann, wie in Figur 2 dargestellt, vorzugsweise zweistufig durchgeführt werden. "K1" bzw. "K2" bedeuten hierbei die für die jeweiligen Stufen verwendeten Kolonnen. Weiterhin ist es bevorzugt, dass in dieser Ausführungsform zusätzlich noch eine Wasserabtrennung durchgeführt wird. Die Wasserabtrennung kann beispielsweise vor oder nach der Ammoniakabtrennung durchgeführt werden, vorzugsweise erfolgt die Wasserabtrennung im Anschluss an die Ammoniakabtrennung.

Nachfolgend wird die Erfindung anhand von Beispielen verdeutlicht.

### Beispiel 1: Synthese von Formaldehydcyanhydrin (FACH)

In einem 10-I-Reaktionsgefäß mit Propellerrührer werden 7000 g (70 mol) Formaldehyd (30-%ig) vorgelegt und mit Natronlauge (1 mol/L) ein pH-Wert von 5,5 eingestellt. Innerhalb von 3,5 Stunden werden 1870 g (68,3 mol) Blausäure (SO₂-Gehalt 30 ppm) über ein beheiztes U-Rohr unterhalb des Rührers gasförmig eindosiert, wobei die Reaktionstemperatur bei 30 °C und der pH-Wert bei 5,5 gehalten werden. Nach 30 Minuten Nachrührzeit wird der pH-Wert mit Schwefelsäure (50-%ig) auf 2,5 gestellt. Über Liebig-Titration wird der FACH-Gehalt ermittelt (ca. 44-%ig).

### Beispiel 2: Synthese von Roh-alpha-Aminoacetonitril (AAN)

Formaldehydcyanhydrin (FACH, ca. 44-%ig, 380,8 g/h, hergestellt nach Beispiel 1) wird mit flüssigem Ammoniak (489 g/h) im molaren Verhältnis 1 : 10 in einem Rohrreaktor (40 ml, Außendurchmesser 3,17 mm) bei 90 °C und 80 bar umgesetzt. Die Verweilzeit im Reaktor beträgt zwei Minuten. Nach dem Reaktor wird das Gemisch auf 10 °C abgekühlt und in einem Flash-Behälter entspannt, wobei überschüssiges Ammoniak teilweise entfernt wird. Der Reaktionsaustrag ist FACH-frei (Bestimmung über Liebig-Titration). Man erhält Roh-AAN als wässrige Lösung (Ausbeute AAN bezogen auf FACH: > 98 %; Verhältnis AAN IDAN: > 99 : 1). Ausbeute und Selektivität werden mittels quantitativer HPLC bestimmt (stationäre Phase: 3 x Atlantis T3, 5 µm, 250 mm x 4,6 mm, Waters; mobile Phase: 50 Vol.-% Wasser / 50 Vol.-% Acetonitril mit 0,5 g/l Ammoniumformiat; Fluss: 0,8 ml/min).

### Beispiel 3: Hydrierung von Roh-AAN

Die kontinuierliche Hydrierung von AAN (hergestellt nach Beispiel 2) wird in einem 270-ml-Autoklaven mit Stromstörern und Scheibenrührer durchgeführt. Dazu werden 10 g einer wässrigen Suspension eines Cr-dotierten Raney-Kobalt-Katalysators (Ra-Co 2724, Fa. Grace) vorgelegt (entsprechend 5 g trockener Katalysator), kontinuierlich 15 NI/h Wasserstoff zudosiert und der Autoklav auf 100 °C gebracht. Bei 180 bar werden dann pro Stunde 18 g einer 27-%igen Roh-AAN-Lösung in Wasser/Ammoniak und 40 g reinem Ammoniak zugefahren. Der Suspensionskatalysator wird durch ein Filterelement aus Sintermetall im Reaktor zurückgehalten.

Über einen Zeitraum von 588 Stunden sinkt die Ausbeute für EDA von 96,1 % auf 91,1 %, während die Ausbeute für DETA von 1,1 % auf 4,1 % ansteigt. Die Me-EDA-Ausbeute liegt konstant bei 3,6 bis 3,7 %.

Analytik: Die Analytik des Reaktionsaustrages erfolgt über GC mit faktorisierten Flächen-%.

| | |
|---|---|
| Säule: | RTX-5 Amine, 30 m, 0,25 mm, 1,0 µm |
| Temperaturprogramm: | 60 °C - 5 min isotherm - 15 °C/min - 280 °C |
| Schwefelgehalt des Katalysators: | 0,2 Atom-% S²⁻ und 0,3 Atom-% S⁶⁺ auf der Oberfläche, bestimmt nach XPS |

### Beispiel 4: Hydrierung von Roh-AAN mit nachfolgender Schwefelzugabe (Vergleich)

Die kontinuierliche Hydrierung von AAN (hergestellt nach Vorschrift von Beispiel 2) wird in einem 270-ml-Autoklaven mit Stromstörern und Scheibenrührer durchgeführt. Dazu werden 10 g einer wässrigen Suspension eines Cr-dotierten Raney-Kobalt-Katalysators (Ra-Co 2724, Fa. Grace) vorgelegt (entsprechend 5 g trockener Katalysator), kontinuierlich 15 NI/h Wasserstoff zudosiert und der Autoklav auf 100 °C gebracht. Der Suspensionskatalysator wird durch ein Filterelement aus Sintermetall im Reaktor zurückgehalten. Bei 180 bar werden dann pro Stunde 18 g einer 27-%igen Roh-AAN-Lösung in Wasser/Ammoniak und 40 g reinem Ammoniak zugefahren. Nach 60 Stunden Laufzeit unter den Bedingungen liegt der EDA-Gehalt im Austrag bei 95,3 %, DETA bei 1,8 % und Me-EDA bei 2,5 %. Ab 60 Stunden Laufzeit wird ca. 50 ppm Schwefel in Form von H₂SO₃ als 6-%ige wässrige Lösung zum Roh-AAN-Feed zugegeben. Nach 40 Stunden unter den Bedingungen sinkt der EDA-Gehalt im Austrag auf 17 % und der Austrag enthält noch 19 % AAN.

Analytik: Die Analytik des Reaktionsaustrages erfolgt über GC mit faktorisierten Flächen-%.

| | |
|---|---|
| Säule: | RTX-5 Amine, 30 m, 0,25 mm, 1,0 µm |
| Temperaturprogramm: | 60 °C - 5 min isotherm - 15 °C/min - 280 °C |

## Patentansprüche

1. Verfahren zur Herstellung von Ethylendiamin (EDA), umfassend die Schritte a) bis c):
a) Umsetzung von Formaldehyd und Blausäure (HCN) zu Formaldehydcyanhydrin (FACH), wobei der Schwefeldioxid-Gehalt in der eingesetzten Blausäure weniger als 100 Gew.-ppm, bezogen auf die Menge an eingesetzter Blausäure, beträgt,
b) Umsetzung von FACH mit Ammoniak (NH₃) zu Aminoacetonitril (AAN),
c) Hydrierung von AAN in Gegenwart eines Katalysators unter Erhalt von EDA.

2. Verfahren gemäß Anspruch 1, **dadurch gekennzeichnet, dass** in Schritt a) das Molverhältnis von HCN zu Formaldehyd 0,85 bis 1,00 zu 1 [Mol/Mol] beträgt und/oder wässriger Formaldehyd mit einem Formaldehydanteil von 20 bis 60 Gew.-% [Mol/Mol] eingesetzt wird.

3. Verfahren gemäß Anspruch 1 oder 2, **dadurch gekennzeichnet, dass** in Schritt b) das Molverhältnis von FACH zu Ammoniak 1 : 2 bis 1 : 50 [Mol/Mol] beträgt.

4. Verfahren gemäß einem der Ansprüche 1 bis 3, **dadurch gekennzeichnet, dass** in Schritt b) der Druck so hoch ist, dass das Reaktionsgemisch flüssig ist, vorzugsweise ist der Druck in Schritt b) höher als in Schritt c).

5. Verfahren gemäß einem der Ansprüche 1 bis 4, **dadurch gekennzeichnet, dass** in Schritt c) ein Raney-Katalysator eingesetzt wird, bevorzugt ein Raney-Nickel- oder ein Raney-Kobalt-Katalysatar, insbesondere ein Raney-Kobalt-Katalysator, der als Promotor mindestens eines der Elemente Fe, Ni oder Cr enthält.

6. Verfahren gemäß einem der Ansprüche 1 bis 5 **dadurch gekennzeichnet, dass** in Schritt c) die Katalysatorbelastung 0,1 bis 3 Gramm AAN pro Gramm Katalysator und Stunde, vorzugsweise 0,2 bis 1,5 Gramm AAN pro Gramm Katalysator und Stunde, insbesondere 0,4 bis 1,2 Gramm AAN pro Gramm Katalysator und Stunde beträgt.

7. Verfahren gemäß einem der Ansprüche 1 bis 6, **dadurch gekennzeichnet, dass** der Katalysator in Schritt c) eine BET-Oberfläche von 10 bis 100 m² pro Gramm Katalysator, vorzugsweise von 20 bis 500 m² pro Gramm Katalysator, insbesondere von 30 bis 100 m² pro Gramm Katalysator, aufweist.

8. Verfahren gemäß einem der Ansprüche 1 bis 7, **dadurch gekennzeichnet, dass** in Schritt c) die Temperatur 20 bis 150 °C und/oder der Druck 40 bis 400 bar beträgt.

9. Verfahren gemäß einem der Ansprüche 1 bis 8, **dadurch gekennzeichnet, dass** das in Schritt b) erhaltene AAN
i) ohne destillative Aufarbeitung und/oder ohne Wasserabtrennung als Roh-AAN in Schritt c) hydriert wird, oder
ii) einer adsorptiven Reinigung unter Verwendung eines Ionentauschers oder eines Metalloxids unterzogen wird, bevor es gemäß Schritt c) hydriert wird.

10. Verfahren gemäß einem der Ansprüche 1 bis 9, **dadurch gekennzeichnet, dass** im Anschluss an die Hydrierung gemäß Schritt c) als Schritt d) eine Ammoniakabtrennung aus dem Hydrierungsprodukt durchgeführt wird.

11. Verfahren gemäß Anspruch 10, **dadurch gekennzeichnet, dass** Schritt d) in zwei Stufen durchgeführt wird, wobei
in der ersten Stufe aus einer Kolonne (K1) Ammoniak über Kopf bei 20 bis 70 °C abgezogen und auskondensiert wird, die Sumpftemperatur kleiner als 220 °C beträgt und aus dem Sumpf ein NH₃-abgereicherter Strom in eine zweite Kolonne (K2) überführt wird,
in der zweiten Stufe in der zweiten Kolonne (K2) EDA aus dem Sumpf abgetrennt wird, wobei das EDA (weitgehend) frei von Ammoniak ist, und gegebenenfalls ein NH₃-haltiger Strom in die Kolonne (K1) rückgeführt wird.

12. Verfahren gemäß Anspruch 10 oder 11, **dadurch gekennzeichnet, dass** Ammoniak aus Schritt d) nach Schritt b) rückgeführt wird.

13. Verfahren gemäß einem der Ansprüche 1 bis 12, **dadurch gekennzeichnet, dass** nach der Hydrierung gemäß Schritt c) Wasser aus dem Hydrierungsprodukt abgetrennt wird, vorzugsweise erfolgt die Wasserabtrennung im Anschluss an die Ammoniakabtrennung gemäß Schritt d).

14. Verfahren gemäß einem der Ansprüche 1 bis 13, **dadurch gekennzeichnet, dass** nach der Hydrierung gemäß Schritt c) EDA destillativ aus dem Hydrierungsprodukt abgetrennt wird, vorzugsweise im Anschluss an die Ammoniakabtrennung gemäß Schritt d) und/oder die Wasserabtrennung.

15. Verfahren gemäß einem der Ansprüche 1 bis 14, daduch gekennzeichnet, dass in Schritt c) ein Raney-Kobalt-Katalysator mit der Zusammensetzung Al: 2-6 Gew.-%, Co: ≥ 86 Gew.-%, Fe: 0-1 Ges.-%, Ni: 1-4 Gew.-%, Cr: 1,5-3,5 Gew.-% eingesetzt wird.

16. Verfahren gemäß einem der Ansprüche 1 bis 15, **dadurch gekennzeichnet, dass** der Schwefeldioxid-Gehalt in der eingesetzten Blausäure weniger als 10 Gew.-ppm, bevorzugt weniger al 1 Gew.-ppm, besonders bevorzugt weniger als 0,1 Gew.-ppm, bezogen auf die Menge an eingesetzter Blausäure, enthält.

## Claims

1. A process for preparing ethylenediamine (EDA), which comprises the steps a) to c):
a) reaction of formaldehyde and hydrocyanic acid (HCN) to form formaldehyde cyanohydrin (FACH), where the Sulfur dioxide content in the hydrocyanic acid used is less than 100 ppm by weight, based on the amount of hydrocyanic acid used,
b) reaction of FACH with ammonia (NH₃) to form aminoacetonitrile (AAN),
c) hydrogenation of AAN in the presence of a catalyst to give EDA.

2. The process according to claim 1, wherein, in step a), the molar ratio of HCN to formaldehyde is from 0.85 to 1.00 : 1 [mol/mol] and/or aqueous formaldehyde having a formaldehyde content of from 20 to 60% by weight [mol/mol] is used.

3. The process according to claim 1 or 2, wherein the molar ratio of FACH to ammonia in step b) is from 1 : 2 to 1 : 50 [mol/mol].

4. The process according to any of claims 1 to 3, wherein the pressure in step b) is so high that the reaction mixture is liquid, and the pressure in step b) is preferably higher than in step c).

5. The process according to any of claims 1 to 4, wherein a Raney catalyst, preferably a Raney nickel catalyst or a Raney cobalt catalyst, in particular a Raney cobalt catalyst comprising at least one of the elements Fe, Ni or Cr as promoter, is used in step c).

6. The process according to any of claims 1 to 5, wherein the space velocity over the catalyst in step c) is from 0.1 to 3 gram of AAN per gram of catalyst an hour, preferably from 0.2 to 1.5 gram of AAN per gram of catalyst an hour, in particular from 0.4 to 1.2 gram of AAN per gram of catalyst an hour.

7. The process according to any of claims 1 to 6, wherein the catalyst in step c) has a BET surface area of from 10 to 100 m² per gram of catalyst, preferably from 20 to 500 m² per gram of catalyst, in particular from 30 to 100 m² per gram of catalyst.

8. The process according to any of claims 1 to 7, wherein, in step c), the temperature is from 20 to 150°C and/or the pressure is from 40 to 400 bar.

9. The process according to any of claims 1 to 8, wherein, the AAN obtained in step b) is
i) hydrogenated, as crude AAN in step c) without prior work-up by distillation and/or without removal of water, or
ii) subjected to an adsorptive purification using an ion exchanger or a metal oxide before being hydrogenated in step c).

10. The process according to any of claims 1 to 9, wherein a removal of ammonia from the hydrogenation product is carried out as step d) after the hydrogenation in step c).

11. The process according to claim 10, wherein step d) is carried out in two stages, with
ammonia being taken off at from 20 to 70°C at the top of a column (K1) and condensed out in a first stage in which the temperature at the bottom is less than 220°C and an NH₃-depleted stream is transferred from the bottom to a second column (K2),
in the second stage, EDA being separated off at the bottom of the second column (K2), where the EDA is (largely) free of ammonia, and an NH₃-comprising stream optionally being recirculated to the column (K1).

12. The process according to claim 10 or 11, wherein ammonia is recirculated from step d) to step b).

13. The process according to any of claims 1 to 12, wherein water is separated off from the hydrogenation product after the hydrogenation in step c); the removal of water is preferably carried out after the ammonia removal as per step d).

14. The process according to any of claims 1 to 13, wherein EDA is separated off from the hydrogenation product by distillation after the hydrogenation in step c), preferably after the ammonia removal as per step d) and/or the removal of water.

15. The process according to any of claims 1 to 14, wherein a Raney cobalt catalyst having the composition Al: 2-6% by weight, Co: ≥ 86% by weight, Fe: 0-1% by weight, Ni: 1-4% by weight, Cr: 1.5-3.5% by weight is used in step c).

16. The process according to any of claims 1 to 15, wherein the sulfur dioxide content in the hydrocyanic acid used comprises less than 10 ppm by weight, preferably less than 1 ppm by weight, particularly preferably less than 0.1 ppm by weight, based on the amount of hydrocyanic acid used.

## Revendications

1. Procédé pour la préparation d'éthylènediamine (EDA), comprenant les étapes a) à c) :
a) transformation de formaldéhyde et d'acide cyanhydrique (HCN) en cyanhydrine de formaldéhyde (FACH), la teneur en dioxyde de soufre dans l'acide cyanhydrique utilisé étant inférieure à 100 ppm en poids par rapport à la quantité de l'acide cyanhydrique utilisé,
b) transformation de FACH avec de l'ammoniac (NH₃), en aminoacétonitrile (AAN),
c) hydrogénation d'AAN en présence d'un catalyseur avec obtention d'EDA.

2. Procédé selon la revendication 1, **caractérisé en ce que**, dans l'étape a), le rapport molaire de HCN à formaldéhyde est de 0,85 à 1,00:1 [moles/moles] et/ou le formaldéhyde aqueux est utilisé en une proportion de formaldéhyde de 20 à 60% en poids [moles/moles].

3. Procédé selon la revendication 1 ou 2, **caractérisé en ce que**, dans l'étape b), le rapport molaire de FACH à ammoniac est de 1:2 à 1:50 [moles/moles].

4. Procédé selon l'une quelconque des revendications 1 à 3, **caractérisé en ce que**, dans l'étape b), la pression présente un niveau tel que le mélange réactionnel est liquide, de préférence, la pression dans l'étape b) est supérieure à celle dans l'étape c).

5. Procédé selon l'une quelconque des revendications 1 à 4, **caractérisé en ce qu'**on utilise, dans l'étape c), un catalyseur de Raney, de préférence un catalyseur à base de nickel de Raney ou de cobalt de Raney, en particulier un catalyseur à base de cobalt de Raney, qui contient comme promoteur au moins un des éléments Fe, Ni ou Cr.

6. Procédé selon l'une quelconque des revendications 1 à 5, **caractérisé en ce que**, dans l'étape c), la charge du catalyseur est de 0,1 à 3 grammes d'AAN par gramme de catalyseur et par heure, de préférence de 0,2 à 1,5 gramme d'AAN par gramme de catalyseur et par heure, en particulier de 0,4 à 1,2 gramme d'AAN par gramme de catalyseur et par heure.

7. Procédé selon l'une quelconque des revendications 1 à 6, **caractérisé en ce que** le catalyseur dans l'étape c) présente une surface BET de 10 à 100 m² par gramme de catalyseur, de préférence de 20 à 500 m² par gramme de catalyseur, en particulier de 30 à 100 m² par gramme de catalyseur.

8. Procédé selon l'une quelconque des revendications 1 à 7, **caractérisé en ce que**, dans l'étape c), la température est de 20 à 150°C et/ou la pression est de 40 à 400 bars.

9. Procédé selon l'une quelconque des revendications 1 à 8, **caractérisé en ce que** l'AAN obtenu dans l'étape b)
i) est hydrogéné en tant qu'AAN brut dans l'étape c) sans traitement par distillation et/ou sans séparation d'eau, ou
ii) est soumis à une purification par adsorption avec utilisation d'un échangeur ionique ou d'un oxyde métallique, avant son hydrogénation selon l'étape c).

10. Procédé selon l'une quelconque des revendications 1 à 9, **caractérisé en ce qu'**après l'hydrogénation selon l'étape c), comme étape d), une séparation d'ammoniac à partir du produit d'hydrogénation est réalisée.

11. Procédé selon la revendication 10, **caractérisé en ce que** l'étape d) est réalisée en deux étapes, de l'ammoniac étant soutiré, dans la première étape, d'une colonne (K1) via à la tête à 20 jusqu'à 70°C et séparé par condensation, la température du fond étant inférieure à 220°C et un flux appauvri en NH₃ étant transféré du fond dans une deuxième colonne (K2), dans la deuxième étape, dans la deuxième colonne (K2), l'EDA étant séparée du fond, l'EDA étant (dans une large mesure) exempte de l'ammoniac et un flux contenant du NH₃ étant éventuellement recyclé dans la colonne (K1).

12. Procédé selon la revendication 10 ou 11, **caractérisé en ce que** l'ammoniac de l'étape d) est recyclé vers l'étape b).

13. Procédé selon l'une quelconque des revendications 1 à 12, **caractérisé en ce qu'**après l'hydrogénation selon l'étape c), l'eau est séparée du produit d'hydrogénation, la séparation de l'eau étant de préférence réalisée après la séparation de l'ammoniac selon l'étape d).

14. Procédé selon l'une quelconque des revendications 1 à 13, **caractérisé en ce qu'**après l'hydrogénation selon l'étape c), l'EDA est séparée par distillation du produit d'hydrogénation, de préférence après séparation de l'ammoniac selon l'étape d) et/ou après la séparation d'eau.

15. Procédé selon l'une quelconque des revendications 1 à 14, **caractérisé en ce qu'**on utilise, dans l'étape c), un catalyseur à base de cobalt de Raney présentant la composition Al : 2-6% en poids, Co : ≥ 86% en poids, Fe : 0-1% en poids, Ni : 1-4% en poids, Cr : 1,5-3,5% en poids.

16. Procédé selon l'une quelconque des revendications 1 à 15, **caractérisé en ce que** la teneur en dioxyde de soufre dans l'acide cyanhydrique utilisé comprend inférieure à 10 ppm en poids, de préférence inférieure à 1 ppm en poids, de manière particulièrement préférée inférieure à 0,1 ppm en poids, par rapport à la quantité d'acide cyanhydrique utilisé.
